## Europäisches Patentamt
## European Patent Office
## Office européen des brevets

(19)

(11) Veröffentlichungsnummer: **0 070 512**
**B1**

(12) **EUROPÄISCHE PATENTSCHRIFT**

(45) Veröffentlichungstag der Patentschrift:
06.06.84

(21) Anmeldenummer: 82106299.9

(22) Anmeldetag: 14.07.82

(51) Int. Cl.³: **C 07 C 85/06**, B 01 J 31/04

(54) **Verfahren zur Herstellung von tert. Aminen.**

(30) Priorität: 22.07.81 DE 3128889

(43) Veröffentlichungstag der Anmeldung:
26.01.83 Patentblatt 83/4

(45) Bekanntmachung des Hinweises auf die Patenterteilung:
06.06.84 Patentblatt 84/23

(84) Benannte Vertragsstaaten:
BE CH DE FR GB IT LI NL

(56) Entgegenhaltungen:
DE - A - 2 625 196
DE - A - 3 005 953
US - A - 3 708 539

Patent Abstracts of Japan Band 5, Nr. 16, 30. Januar 1981

(73) Patentinhaber: BASF Aktiengesellschaft,
Carl-Bosch-Strasse 38, D-6700 Ludwigshafen (DE)

(72) Erfinder: Mueller, Herbert, Dr., Carostrasse 53,
D-6710 Frankenthal (DE)
Erfinder: Axel, Hartmut, Dr., Bahnhofsanlage 32,
D-6830 Schwetzingen (DE)
Erfinder: Wittwer, Arnold, Dr., Londoner Ring 61,
D-6700 Ludwigshafen (DE)

**Beschreibung**

Die katalytische Alkylierung von Ammoniak oder Aminen mit Alkoholen ist bekannt. Bei dieser Reaktion werden einerseits dehydratisierende Oxide, z. B. die des Aluminiums, Thoriums, Wolframs und Chroms als Katalysatoren verwendet, andererseits sind Hydrierungs- bzw. Dehydrierungskatalysatoren mit den aktiven Metallen Kupfer, Nickel und Cobalt oder Edelmetallen empfohlen worden. Die Hydrierungs/Dehydrierungskatalysatoren sind Feststoffe. Sie werden in Form einer Suspension, wenn sie in Pulverform vorliegen oder als Formkörper im fest angeordneten Bett angewendet. Es wurden Verfahren in flüssiger Phase sowie in der Gasphase vorgeschlagen. In einer Arbeit von V. A. Nekrasova und N. I. Shuikin in der Zeitschrift Russian Chemicals Reviews, 34, 843 (1965) sowie in dem Buch »The acyclic aliphatic tertiary Amines«, L. Spialter a. J. A. Pappalardo The Macmillan Comp., 1965, ist eine ausführliche Darstellung des Sachgebietes abgehandelt. Besonders schwierig ist die Herstellung von tertiären Aminen durch Alkylierung von sekundären Aminen mit Alkoholen. Als Folge von Transalkylierungsreaktionen treten sekundäre und primäre Amine als Nebenprodukte auf, die nur schwer aus den gewünschten Reaktionsprodukten abzutrennen sind und bei der Anwendung der gewünschten tertiären Amine stören können.

Deshalb wurde, um tertiäre Amine mit hohen Ausbeuten herstellen zu können, in der DE-OS 1 493 781 vorgeschlagen, daß mindestens stöchiometrische Mengen des sekundären Amins mit dem Alkohol umgesetzt werden, da ein Überschuß an Alkohol nachteilig sein soll. Zwar scheint man bei der Umsetzung von sekundären Alkoholen mit dieser Maßnahme eine gewisse Verbesserung der Selektivität des Amins zu erreichen, der Umwandlungsgrad des Amins ist allerdings gering. Bei primären Alkoholen werden jedenfalls nur geringe Umsätze und eine schlechte Selektivität erzielt; denn als Nebenprodukte entstehen Rückstände, die die Ausbeute vermindern.

Ein verbessertes Verfahren zur Herstellung von tertiären Aminen beschreibt die US-Patentschrift 3 708 539. Hier wird in flüssiger Phase ein Alkohol mit einem sekundären Amin an Ruthenium-, Osmium-, Rhenium- oder Technetium-Katalysatoren umgesetzt. Nachteilig bei diesem Verfahren ist vor allem der Umstand, daß Umsatz und Ausbeute bezogen auf die eingesetzten Alkohole gemessen am Wert der Katalysator-Rohstoffe, für ein technisches Verfahren unzureichend sind.

Nach der US-Patentschrift 3 223 734 kann man Raney-Nickel, Kupfer-Chromoxid, Palladium auf Kohle oder Nickel auf Diatomeen-Erde als Katalysator zur Herstellung von tertiären Aminen verwenden. Wie die Beispiele de Patentschrift zeigen, sind jedoch auch mit diesem Katalysatoren keine günstigen Ergebnisse zu erzielen und es werden auch oft unwirtschaftlich große Mengen gebraucht.

Eine wesentlich verbesserte Methode zur Herstellung von Dimethyldodecylamin wird in der japanischen Offenlegungsschrift 19 604/77 beschrieben. Man erhält Dimethyldodecylamin mit einem Kupfer-Chromoxid-Katalysator auf Diatomeen-Erde mit einer Ausbeute um 90%.

Bei den Katalysatoren in den vorerwähnten Patentveröffentlichungen handelt es sich um feste (heterogene) Katalysatoren mit einer verhältnismäßig geringen spezifischen Aktivität. Man ist daher gezwungen, hohe Mengen (z. B. 2 bis 10% Katalysator, bezogen auf Reaktionsgemisch) einzusetzen. Obwohl es ein Vorteil der Feststoffe ist, daß sie nach der Umsetzung leicht durch Filtration oder Sedimentation vom Substrat getrennt werden können, sind doch erhebliche technische und wirtschaftliche Probleme zu bewältigen. Bei einer Katalysatorkonzentration von über 5% treten z. B. bei Suspensionsverfahren Durchmischungsprobleme auf. Außerdem stellt die Beseitigung bzw. Aufarbeitung der durch organische Bestandteile verunreinigten inaktiv gewordenen Katalysatoren besondere Anforderungen, wenn es sich um Trägerkatalysatoren handelt oder wenn sie giftige Bestandteile (Chromate) zu ihrer Herstellung benötigen. Überdies befriedigen diese Katalysatoren auch im Hinblick auf ihre Selektivität nicht völlig.

Deshalb wird in den deutschen Offenlegungsschriften 2 907 869 und 3 005 953 ein quasihomogenes, kolloidales Katalysatorsystem vorgeschlagen, das die beschriebenen Nachteile der festen Katalysatoren nicht aufweist. Diese kolloidalen Katalysatoren entstehen bei der Reduktion einer Mischung carbonsaurer Salze von Kupfer oder Silber mit carbonsauren Salzen von Elementen der Gruppe VIII des Perioden-Systems (einschließlich Mangan und Zink) sowie carbonsauren Salzen der Alkali- und Erdalkali-Elemente mit Wasserstoff bzw. Aluminiumalkylverbindungen. Statt Salzen von Carbonsäuren können auch innere Komplexe von z. B. Dicarbonylverbindunngen verwendet werden.

Bei diesen Katalysatoren erreicht man, wie auch sonst von Aminolysen allgemein bekannt, gute Aktivität und Selektivität nur, wenn eine Kombination aus mehreren aktiven Metallen in den richtigen Mischungsverhältnissen angewendet wird. Von Nachteil ist auch, daß die Katalysatoren vor der eigentlichen Umsetzung durch Reduktion mit Wasserstoff oder Aluminiumalkylen aktiviert werden müssen. Schließlich können die Katalysatoren nicht auf einfache Art und Weise mechanisch abgetrennt werden, sondern sollen durch Destillation vom Reaktionsprodukt getrennt werden. Dadurch verbleibt der Katalysator in den gleichzeitig entstandenen höhersiedenden oder undestillierbaren Rückständen. Die Katalysatoren können zwar zunächst für eine weitere Umsetzung eingesetzt werden, es läßt sich aber nicht vermeiden, daß sich dadurch der Nebenproduktspiegel im Reaktionssystem mehr und mehr erhöht. Abgesehen davon, daß empfindliche Substanzen die Gegenwart katalytisch wirkender Metalle bei der Destillation schlecht überstehen und Veränderungen erleiden können, verbleibt noch das Problem, diese Katalysatoren zu einem späteren Zeitpunkt aus den Rückständen umweltfreundlich abzutrennen.

Es ist daher eine Aufgabe der Erfindung, aliphatische bzw. cycloaliphatische tertiäre Amine mit Mitteln herzustellen, bei denen eine hohe Ausbeute der Zielprodukte, möglichst frei von primären und sekundären Aminen mit einem minimalen Katalysatoreinsatz erreicht wird. Außerdem soll eine möglichst vollständige Umsetzung der Alkohole stattfinden, da die spätere Abtrennung der Alkohole von den Zielprodukten oft schlecht gelingt. Der Siedepunkt der Alkohole und der entsprechenden Amine liegt nämlich insbesondere bei den höhermolekularen sogenannten Fettaminen so dicht beisammen, daß eine Trennung durch Destillation mißlingt. Der Katalysator soll schließlich nach der Umsetzung von einer solchen Beschaffenheit sein, daß er leicht quantitativ vom Reaktionsprodukt abgetrennt und für weitere Umsetzungen verwendet werden kann. Schließlich sollten Rückstände, höhermolekulare Kondensationsprodukte und Nebenreaktionen weitgehend ausgeschlossen werden. Der Katalysator für eine derartig ausgezeichnete Umsetzung muß darüber hinaus eine einfache, leicht zugängliche wohlfeile Chemikalie sein, die möglichst ohne einen eigenen Aktivierungsschritt unmittelbar für die Umsetzung wirksam wird.

Es wurde gefunden, daß man symmetrische oder unsymmetrische tertiäre Amine mit insgesamt bis zu etwa 40 Kohlenstoffatomen durch Umsetzung sekundärer Amine mit gleichen oder verschiedenen Substituenten, mit primären oder sekundären ein- oder mehrwertigen Alkoholen, insbesondere Alkoholen, die 6 oder mehr Kohlenstoffatome besitzen und sich wie ein Fettalkohol verhalten, an einem Kupferkatalysator gegebenenfalls in der Gegenwart von Wasserstoff, vorteilhaft erhält, wenn man einen Katalysator verwendet, wie er aus Kupferformiat unter den Reaktionsbedingungen von selbst entsteht.

Dabei wird vorteilhaft in an sich bekannter Weise so verfahren, daß man das jeweilige Amin zum flüssigen, den jeweiligen Alkohol enthaltenden Reaktionsgemisch im Maße der Umsetzung zuführt und Wasser im Maße seiner Entstehung entfernt.

Die Aufgabe wird somit am besten dadurch gelöst, daß man den Alkohol und das Amin in Gegenwart geringer Mengen Kupferformiat in einer Weise zusammenbringt, daß das Amin sich in starkem Unterschuß befindet und der Alkohol im wesentlichen die flüssige Phase bildet. Die flüssige Phase enthält folglich den Alkohol, jedenfalls zu Beginn der Reaktion im hohen molaren Überschuß. Im zeitlichen Gleichgewicht sollte das Amin eine Menge von z. B. 10 Gew.-%, bezogen auf das Reaktionsgemisch, nicht überschreiten. Der Katalysator entfaltete unter diesen Bedingungen ohne vorhergehende zusätzliche Aktivierung seine maximale katalytische Wirkung.

Hierzu wird am einfachsten so verfahren, daß man das umzusetzende Amin dem flüssigen Reaktionsgemisch in Dampfform zuführt, wobei man von einem Reaktionsgemisch ausgeht, das bereits zu Beginn der Umsetzung geringe Mengen Amin (bis zu 3 Gew.-%), das Kupferformiat und den Alkohol enthält. Beim Erreichen einer Temperatur von im allgemeinen etwa 150°C setzt dann die beabsichtigte Umsetzung von selbst ein.

Es ist möglich, das umzusetzende Amin flüssig, z. B. über Dosiereinrichtungen zuzusetzen, wobei sich dieses Verfahren dann anbietet, wenn die Reaktionstemperatur über der Siedetemperatur des Amins liegt und die Reaktion isobar geführt wird. Auch hierdurch ist die Ansammlung größerer oder gar überschüssiger Aminmengen vermeidbar.

Die vorstehenden Erläuterungen bedeuten, daß man bei absatzweiser Durchführung die Umsetzung jeweils unter allmählicher Zugabe des Amins bis zum Verbrauch des Alkohols führt und aus dem flüssigen Reaktionsgemisch das gewünschte Amin durch Destillation gewinnen kann.

In sinngemäßer Abwandlung wird man bei fortlaufender Betriebsweise das umzusetzende Amin zum Beispiel gasförmig im Gegenstrom zu dem den Alkohol und suspendierten Katalysator sowie gegebenenfalls das tertiäre Amin enthaltenden flüssigen Reaktionsgemisch führen. Gegebenenfalls sollte man hier eine Nachreaktionsstrecke vorsehen.

Eine wichtige Maßnahme zur Erzielung optimaler Ergebnisse besteht darin, das gebildete Wasser laufend aus dem Reaktionsgemisch zu entfernen, woraus sich ergibt, daß man vorteilhaft unter Bedingungen arbeitet, bei denen Wasser freiwillig als Gas das Reaktionsgemisch verläßt.

Zum Verständnis der Erfindung kann man sich folgendes vorstellen: findet die Reaktion in der Gasphase statt, so muß eine sehr hohe Reaktionstemperatur gewählt werden, da die Alkohole im allgemeinen relativ schwer flüchtig sind; man erhält dann eine geringe Ausbeute und unreine Produkte. Wird andererseits in flüssiger Phase gearbeitet, indem man z. B. — bei leichtflüchtigen Aminen wie Dimethylamin — hohen Druck unter Berücksichtigung der Temperatur und eine hohe Konzentration an Amin anwendet, so ist der Reaktionsverlauf in hohem Maße unspezifisch. In diesem Sinne wirkt auch die mit dem Umsatz gewöhnlich zunehmende Wassermenge.

Zweckmäßig hält man die Reaktionsbedingungen ein, wie sie in der DE-OS 2 625 196 beschrieben werden. Durch die Anwendung von Kupferformiat als Katalysator gelingt es dabei mit wesentlich geringeren Katalysatormengen auszukommen, ein reineres Produkt zu erhalten und die Ausbeute weiter zu erhöhen.

Vorteilhaft ist es weiterhin, in Gegenwart von z. B. 0,1 bis 50 Gew.-%, vorzugsweise 1 bis 10 Gew.-% (bezogen auf den Kupferkatalysator) einer Base, etwa Alkali- oder Erdalkalihydroxid, z. B. Natriumhydroxid, Kaliumhydroxid oder Calciumhydroxid sowie besonders vorteilhaft den entsprechenden Carbonaten zu arbeiten, wobei mehrere der besagten Verbindungen gleichzeitig anwesend sein können.

Die Reaktion kann an sich — entsprechend der Brutto-Umsatzgleichung — in Abwesenheit von Wasserstoff durchgeführt werden. So schreitet die Reaktion beispielsweise zwischen einem Alkohol und Dimethylamin allein durch Zuführung von Dimethylamingas zur Reaktionsmischung fort, sobald die notwendige Reaktionstemperatur erreicht ist. Man erhält das gewünschte Amin in einer Ausbeute von über 95% bei einer Alkoholumwandlung von über 99%. Dennoch hat es sich in manchen Fällen als vorteilhaft erwiesen, geringe Mengen Wasserstoff dem Reaktionsmilieu zuzuführen, was der Aktivität des Katalysators förderlich ist und verhindert, daß geringe Mengen ungesättigte Verbindungen entstehen.

An die Beschaffenheit und die Eigenschaften des Kupferformiats werden keine besonderen Anforderungen gestellt. Man kann z. B. handelsübliches kristallwasserhaltiges Kupferformiat ohne Vorbehandlung einsetzen. Mit demselben Erfolg läßt sich aber auch wasserfreies Kupferformiat verwenden. Die Art und Weise, wie das Kupfersalz hergestellt wurde, ist für dessen katalytische Aktivität ohne Bedeutung. Beispielsweise erhält man einen geeigneten Katalysator, wenn man das Formiat aus dem Kupferoxid, -hydroxid, -stearat oder -carbonat und Ameisensäure herstellt. Diese Umsetzung kann auch in Abwesenheit von Wasser z. B. in dem für die Aminolyse vorgesehenen Alkohol mit der Kupferverbindung und Ameisensäure erfolgen. Es kann angenommen werden, daß sich der eigentliche Katalysator durch die Umsetzung von Kupferformiat mit Bestandteilen des Reaktionsgemisches von selbst bildet. Die Menge an Kupferformiat zur Erzielung ausreichenden Umsatzes beträgt 0,01 bis 2 Gew.-%, vorzugsweise 0,05 bis 1 Gew.-% (berechnet als metallisches Kupfer), bezogen auf den Ausgangsalkohol. Da sich die aus dem Kupferformiat entstehenden Katalysatoren bei Verwendung von reinen Ausgangsmaterialien praktisch nicht verbrauchen und wiederverwendet werden können, spielen dessen geringe Kosten keine Rolle und sie können irgendwann auch abgetrennt und wieder in Kupferformiat umgewandelt und gegebenenfalls nach erneuter Zugabe einer basischen Alkali- oder Erdalkalimetallverbindung erneut verwendet werden.

Der als Ausgangsmaterial verwendete Alkohol kann einen linearen oder verzweigten, gesättigten oder ungesättigten, aliphatischen Kohlenwasserstoffrest besitzen, dessen Kohlenstoffkette durch Heteroatome, insbesondere Sauerstoffatome unterbrochen sein kann. Obwohl auch sekundäre Alkohole nach dem vorliegenden Verfahren umgesetzt werden können, wird die Reaktion mit primären Alkoholen bevorzugt. Die besten Ergebnisse werden also mit mono- und polyfunktionellen primären Alkoholen erhalten, deren Anzahl an Kohlenstoffatomen 2 oder mehr beträgt.

Technisch besonders wichtige Zielprodukte sind die sogenannten Fettamine; sie leiten sich von Alkoholen ab, die etwa 6 bis 22 Kohlenstoffatome im Molekül aufweisen (sogenannte Fettalkohole). Genannt seien Octylalkohol, Laurylalkohol, Myristylalkohol, Cetylalkohol, Oleylalkohol, Stearylalkohol, Tridecylalkohol, Pentadecylalkohol oder Gemische dieser Verbindungen. Ferner eignen sich z. B. zweiwertige Alkohole wie Ethylenglykol, Diethylenglykol, Butandiol-1,4 sowie Hexandiol-1,6. Bei Alkoholen, die weniger als etwa 6 Kohlenstoffatome enthalten, werden bevorzugt die mehrwertigen schwerflüchtigen Alkohole umgesetzt.

Anstelle der Alkohole können auch lineare und verzweigte, gesättigte und ungesättigte, aliphatische Aldehyde, wie Laurylaldehyd oder auch Oxoaldehyde verwendet werden, die in Gegenwart von Wasserstoff unter den Reaktionsbedingungen zu Alkoholen reduziert werden.

Sekundäre Amine, die für das erfindungsgemäße Verfahren geeignet sind, besitzen gleiche oder verschiedene aliphatische oder cycloaliphatische Substituenten mit einer Kohlenstoffzahl von jeweils z. B. 1 bis 18 oder mehr, vorzugsweise 1 bis 16 und insbesondere 1 bis 12. Technisch wichtige sekundäre Amine sind etwa Dimethylamin, Diethylamin, Methylethylamin, Dipropylamin oder Dioctylamin.

Die Reaktion findet bei einer Temperatur zwischen i. a. 150 und 250°C, vorzugsweise zwischen 190 und 220°C statt. Sie kann unter atmosphärischem, erhöhtem oder erniedrigtem Druck ausgeführt werden, beispielsweise in einem Bereich von 10 mbar bis 6 bar, wobei der zu wählende Druckbereich auch von den Eigenschaften der beteiligten Reaktionspartner und der gewählten Reaktionstemperatur abhängt.

Verwendet man für die Alkylierung einen hochsiedenden Alkohol, beispielsweise Decanol, Laurylalkohol oder Tridecylalkohol, so genügt es, den Alkohol in Gegenwart des Kupferformiats und gegebenenfalls unter Zusatz der aktivierenden Alkaliverbindung zu erhitzen und das sekundäre Amin im Maße wie es reagiert, einzuleiten. Das bei der Umsetzung gebildete Wasser wird aus dem Reaktionssystem laufend abdestilliert. Dieser letzte Vorgang kann dadurch gefördert werden, daß man mit einem geeigneten Lösungsmittel, etwa einem aliphatischen oder aromatischen Kohlenwasserstoff, das Reaktionswasser als Azeotrop auskreist und abtrennt. Als Reaktionsraum ist deshalb z. B. ein Reaktor geeignet, der mit einer Rührvorrichtung, einem Kondensator und einem Wasserabtrenngefäß ausgerüstet ist. Eine besonders einfache Methode besteht nun darin, durch den auf Reaktionstemperatur gebrachten Alkohol das Amin und falls gewollt Wasserstoff als Gas durchperlen zu lassen. Dabei wird das entstandene Wasser dampfförmig aus der Reaktionszone entfernt, kondensiert und das unverbrauchte Gas wieder zurückgeleitet. Je nach Reaktionstemperatur und Menge des Katalysators kann man stündlich bis zur äquimolaren Menge, vorzugsweise 0,3 bis 0,6 Mol des Amins pro Mol Alkohol in das Reaktionsgemisch einleiten. Die gegebenenfalls zu verwendende Wasserstoffmenge liegt entweder in der gleichen Größenordnung oder auch darunter. Sie ist auf jeden Fall in weiten Grenzen variabel. Aus dem vorstehenden erkennt man, daß man günstige Reaktionsbedingungen dann gewählt hat, wenn es gelingt, die Umsetzung etwa in 1 bis 10 Stunden auszuführen. Kürzere Reaktionszeiten sind wegen der aufzubringenden Wärmemenge für das zu verdampfende Reaktionswasser nicht zu empfehlen. Dagegen können längere Reaktionszeiten ohne Nachteil für die Umsetzung in Kauf genommen werden. Die Reaktionsgeschwindigkeit wird darüber hinaus natürlich immer von der pro Zeiteinheit zugegebenen Menge an Amin bestimmt. Normalerweise wird man sie so wählen, daß die Konzentration an sekundärem Amin gleichbleibend bei einigen Prozent von Reaktionsgemisch liegt.

Ein besonderes Ergebnis der Erfindung ist es, daß das Reaktionsprodukt nach vollständiger Umsetzung und mechanischer Abtrennung des Katalysators ohne weitere Reinigungsoperation für die meisten Einsatzgebiete eine genügende Reinheit aufweist. Die Produkte sind durchweg farblos und sind meist mit weniger als 5% Nebenprodukten verunreinigt.

In zahlreichen Fällen z. B. werden die teriären Amine nicht als solche, sondern als quartäre Salze benutzt, nachdem man sie z. B. mit Benzylchlorid oder Methylchlorid zur Reaktions gebracht hat. Obwohl die Qualitätserfordernisse für z. B. Dimethyllauryl- oder Dimethylmyristylamin sehr streng sind, können die nach der Erfindung hergestellten tertiären Amine ohne Destillation dem gewünschten Einsatzzweck zugeführt werden. Da es sich bei diesen tertiären Aminen um sehr hochsiedende Verbindungen handelt, bedeutet der Wegfall der destillativen Reinigung einen nicht zu vernachlässigende Energieersparung. Gegenüber der Verfahrensweise wie sie in der DE-OS 2 907 869 beschrieben ist, ist außer den bereits mitgeteilten Vorteilen zu erwähnen, daß die Alkylierung bei merklich tieferen Temperaturen eintritt, im Durchschnitt liegt die Reaktionstemperatur im erfindungsgemäßen Verfahren 10 bis 20°C niedriger. Nicht zuletzt dadurch erklärt sich übrigens die nach dem erfindungsgemäßen Verfahren erzielte hohe Selektivität. Da es unter anderem ein Ziel des erfindungsgemäßen Verfahrens ist, den Alkohol vollständig umzusetzen, wird man das Amin insgesamt mindestens in stöchiometrischer Menge anbieten. Von einem leichtflüchtigen Amin kann ein gewisser Überschuß gegen Reaktionsende zweckmäßig sein. Dieser mag im Durchscnnitt zwischen 5 und 100 Mol-%, bevorzugt 10 bis 30 Mol-%, betragen, d. h. man hält gegebenenfalls die Umsatzbedingungen so lange ein, bis ein entsprechender Umsatz erwartet werden kann. Das überschüssig angebotene Amin wird dann wieder entfernt. Es ist wichtig, daß das Amin im Verlaufe der Umsetzung in dem Maße zugegeben wird, wie es sich umsetzt. Dadurch wird erfindungsgemäß erreicht, daß es praktisch während der gesamten Reaktionsdauer auf einen Überschuß an Alkohol trifft, d. h. so lange dieser noch in nennenswerter Konzentration im Reaktionsgemisch vorhanden ist. Solche Reaktionsbedingungen sind sehr einfach bei einem Gegenstromverfahren zu erreichen, das darüber hinaus in fortlaufender Betriebsweise ausgeübt werden kann.

Beispiel 1

In einer Rührapparatur werden bei Normaldruck 1600 kg eines Gemisches aus n-Dodecanol und n-Tetradecanol (70 : 30), 28,5 kg Kupferformiat $Cu(CHO_2)_2 \cdot 4 H_2O$ und 3 kg Calciumhydroxyd vorgelegt und auf 200°C erwärmt. Während der Aufheizperiode (20 Minuten) hält man das Reaktionsgemisch an Wasserstoff und Dimethylamin gesättigt. Oberhalb von 170°C setzt die Reaktion ein, und in einem als Wasserauskreiser ausgebildeten Destillationsaufsatz scheidet sich eine wäßrige Phase ab. Gleichzeitig werden geringe Anteile des vorgelegten Alkohols mitgerissen, die über den Wasserauskreiser in die Rührapparatur zurückgeführt werden. Nun werden stündlich dem Reaktionsgemisch ca. 30 bis 35 kg Dimethylamin und 5 $Nm^3$ Wasserstoff gasförmig zugeführt. Am Anfang der Reaktion destillieren stündlich ca. 12 bis 15 kg Reaktionswasser ab und werden im Wasserabscheider als wäßrige Phase abgetrennt. Mitgerissener Alkohol geht dagegen in den Reaktor zurück. Nach 8 bis 10 Stunden Reaktionszeit kommt die Umsetzung zum Stillstand, es scheidet sich kein Wasser mehr ab. Man läßt nun noch 2 Stunden unter denselben Bedingungen weiterlaufen und unterbricht dann die Zufuhr an Amin. Man läßt den Katalysator 1 Stunde lang absitzen und trennt dann das Reaktionsprodukt durch Filtration von Resten (ca. 3% des Einsatzes) nicht abgesetzten feinen Katalysators. Man erhält ein farbloses Filtrat, das ohne Rektifikation durch Destillation aufgearbeitet wird. Man erhält ein Gemisch von Dimethyllaurylamin und Dimethylmyristylamin (70 : 30) in einer Ausbeute von 96 bis 98% der berechneten Menge. Die Reinheit, gemessen am Gehalt an tertiärem Amin ist höher als 99,5%. Das Amingemisch siedet bei 5 mbar zwischen 118 und 145°C, als Destillationsrückstand verbleiben 2 bis 3%, bezogen auf die Gewichtsmenge des eingesetzten Alkohols, eines halbfesten Rückstandes.

Ein vergleichbares Ergebnis erzielt man auch, wenn Calciumhydroxid durch Bariumhydroxid oder Kaliumcarbonat ersetzt wird. Durch Anwendung von Natriumcarbonat und Natriumbicarbonat erreicht man eine um ca. 20% erhöhte Reaktionsgeschwindigkeit. Die Ausbeute liegt dann bei ca. 95%. Eine etwa — um ca. 10 bis 15% — verringerte Reaktionsgeschwindigkeit wird gemessen, wenn statt des Calciumhydroxidzusatzes 0,1 Teile Kaliumhydroxid oder 0, 3 Teile Calciumoxid zugesetzt werden.

Führt man die Reaktion ohne Zusatz einer Base durch, so erhält man zwar einen vollständigen Alkoholumsatz, jedoch erst nach etwa 30 Stunden. Die Ausbeute beträgt aber auch dann noch 90 bis 92% der berechneten Menge.

Während in Gegenwart von Alkali der Anteil an sekundären und primären Aminen im Zielprodukt praktisch nicht nachweisbar ist, entstehen in Abwesenheit von Alkali ca. 0,2 bis 0,3% primäres Amin und etwa 0,1% sekundäres Amin anderer Art als das zugeführte (Umalkylierung).

### Beispiel 2

Verwendet man bei der Umsetzung statt Dodecanol/Tetradecanol die geradkettigen Alkohole Actanol, Decanol oder Octadecanol, so erhält man dasselbe gute Ergebnis.

Keinen Unterschied findet man, wenn man anstelle der Alkohole die Aldehyde verwendet, also statt des Gemisches Laurylalkohol/Myristilalkohol die davon abgeleiteten Kohlenwasserstoff-Aldehyde wie Laurylaldehyd oder Myristilaldehyd eingesetzt. Die stündlich dem Reaktor zugeleitete Wasserstoffmenge beträgt dann 30 Normalkubikmeter.

### Beispiel 3

Man verfährt wie vorstehend beschrieben, legt jedoch 2000 kg eines $C_{13}/C_{15}$-Oxo-Alkoholgemisches, das durch Hydroformylierung eines Ziegler-Olefin-Gemisches (etwa 70% Dodecen-1 und 30% Tetradecen-1) erhalten wurden, 35,5 kg Kupferformiat sowie 2 kg Calciumhydroxid vor und setzt bei 200°C mit Dimethylamin und Wasserstoff (Wasserstoffpartialdruck ca. 100 mbar) um. Die Umsetzung ist nach 24 Stunden beendet. Man läßt abkühlen, filtriert und erhält etwa 2040 kg Reaktionsprodukt. Es wird durch Destillation aufgearbeitet. Dabei erhält man als Destillat 1885 kg $C_{13}/C_{15}$-Dimethylamin ($Kp_{5\,mbar}$ = 130 bis 154°C; die Aminzahl beträgt 229 mg KOH/g, was in guter Übereinstimmung zur Hydroxyzahl von 250 mg KOH/g des eingesetzten Alkoholgemisches steht). Als Rückstand verbleiben 155 kg eines höhersiedenden Amingemisches.

### Patentansprüche

1. Verfahren zur Herstellung von symmetrischen oder unsymmetrischen tertiären Aminen mit insgesamt bis zu etwa 40 Kohlenstoffatomen durch Umsetzung sekundärer Amine mit gleichen oder verschiedenen Substituenten, mit primären oder sekundären ein- oder mehrwertigen Alkoholen, insbesondere Alkoholen, die 6 oder mehr Kohlenstoffatome besitzen und sich wie ein Fettalkohol verhalten, an einem Kupferkatalysator bei Temperaturen von 150 bis 250°C, gegebenenfalls in Gegenwart von Wasserstoff, dadurch gekennzeichnet, daß man einen Katalysator verwendet, wie er aus Kupferformiat unter den Reaktionsbedingungen von selbst entsteht.

2. Verfahren nach Anspruch 1, dadurch gekennzeichnet, daß man die Umsetzung in Gegenwart eines basischen Alkali- oder Erdalkalimetallverbindung vornimmt.

3. Verfahren nach einem der Ansprüche 1 oder 2, dadurch gekennzeichnet, daß man das sekundäre Amin dem flüssigen, den Alkohol enthaltenden Reaktionsgemisch im Maße der Umsetzung zuführt und Wasser nach seiner Entstehung entfernt.

4. Verfahren nach einem der Ansprüche 1 bis 3, dadurch gekennzeichnet, daß man ein leichtflüchtiges sekundäres Amin in Dampfform zuführt.

5. Verfahren nach einem der Ansprüche 1 bis 4, dadurch gekennzeichnet, daß man das sekundäre Amin dem Reaktionsgemisch bei einer Reaktionstemperatur oberhalb seiner Siedetemperatur zuführt und die Reaktion bei konstantem Druck ablaufen läßt.

6. Verfahren nach einem der Ansprüche 1 bis 5, dadurch gekennzeichnet, daß man die Umsetzung bei absatzweiser Durchführung jeweils bis zum Verbrauch des Alkohols führt und aus dem flüssigen Reaktionsgemisch das Amin durch Destillation gewinnt.

7. Verfahren nach einem der Ansprüche 1 bis 5, dadurch gekennzeichnet, daß man bei fortlaufender Betriebsweise ein leichtflüchtiges sekundäres Amin gasförmig im Gegenstrom zu dem Alkohol und gegebenenfalls tertiäres Amin enthaltenden flüssigen Reaktionsgemisch führt und gegebenfalls eine Nachreaktionsstrecke vorsieht.

8. Verfahren nach einem der Ansprüche 1 bis 7, dadurch gekennzeichnet, daß der vom Reaktionsprodukt abgetrennte Katalysator für weitere Umsetzungen gegebenenfalls nach erneuter Zugabe einer basischen Alkali- oder Erdalkalimetallverbindung erneut verwendet wird.

### Claims

1. A process for the preparation of a symmetrical or asymmetrical tertiary amine having a total of not more than about 40 carbon atoms, by reacting a secondary amine having identical or different substituents with a primary or secondary monohydric or polyhydric alcohol, in particular an alcohol which has 6 or more carbon atoms and behaves like a fatty alcohol, over a copper catalyst, at from 150 to 250°C, in the presence or absence of hydrogen, wherein the catalyst used is one which forms of its own accord from copper formate under the reaction conditions.

2. A process as claimed in claim 1, wherein the reaction is carried out in the presence of a basic alkali metal compound or alkaline earth metal compound.

3. A process as claimed in claim 1 or 2, wherein the secondary amine is fed, at the rate at which it reacts, into the liquid, alcoholcontaining reaction mixture, and water is removed at the rate at which it is formed.

4. A process as claimed in any of claims 1 to 3, wherein a readily volatile secondary amine is fed in in vapor form.

5. A process as claimed in any of claims 1 to 4, wherein the secondary amine is fed into the reaction mixture at above its boiling point, and the reaction is carried out under a constant pressure.

6. A process as claimed in any of claims 1 to 5, wherein, in batch operation, the reaction is carried out until the alcohol is consumed, and the amine is obtained from the liquid reaction mixture by distillation.

7. A process as claimed in any of claims 1 to 5, wherein, in continuous operation, a readily volatile secondary amine, in the form of a gas, is fed counter-current to the liquid reaction mixture which contains the alcohol and may or may not contain a tertiary amine, and a zone for further reaction may be provided.

8. A process as claimed in any of claims 1 to 7, wherein the catalyst which has been separated off from the product is reused for further reactions, if necessary after the addition of a further amount of a basic alkali metal compound or alkaline earth metal compound.

**Revendications**

1. Procédé de préparation d'amines tertiaires symétriques ou non symétriques avec au total jusqu'à 40 atomes de carbone environ par réaction d'amines secondaires avec des substituants identiques ou différents, à des températures de 150 à 250° C et sur un catalyseur au cuivre, avec des alcools primaires ou secondaires mono- ou polyvalents, en particulier des alcools en $C_6$ et plus, qui se comportent comme des alcools gras, éventuellement en présence d'hydrogène, caractérisé en ce que l'on emploie un catalyseur formé in situ, dans les conditions réactionnelles, à partir du formiate de cuivre.

2. Procédé suivant la revendication 1, caractérisé en ce que la réaction est réalisée en présence d'un dérivé de métal alcalin ou alcalinoterreux basique.

3. Procédé suivant l'une des revendications 1 et 2, caractérisé en ce que l'on ajoute l'amine secondaire au mélange réactionnel liquide, contenant l'alcool, au fur et à mesure de la transformation et on élimine l'eau au für et à mesure de sa formation.

4. Procédé suivant l'une des revendications 1 à 3, caractérisé en ce que l'on ajoute une amine secondaire à bas point d'ébullition à l'état gazeux.

5. Procédé suivant l'une des revendications 1 à 4, caractérisé en ce que l'on ajoute l'amine secondaire à une température supérieure à son point d'ébullition au mélange réactionnel, la réaction se déroulant à pression constante.

6. Procédé suivant l'une des revendications 1 à 5, caractérisé en ce que, dans le mode de réalisation discontinu, on laisse la transformation se poursuivre jusqu'à la consommation de l'alcool, puis on extrait l'amine par une distillation du mélange réactionnel liquide.

7. Procédé suivant l'une des revendications 1 à 5, caractérisé en ce que, dans un mode de réalisation continu, on fait circuler une amine secondaire à bas point d'ébullition à l'état gazeux en contre-courant de l'alcool et du mélange réactionnel, contenant éventuellement de l'amine tertiaire, et on prévoit, le cas échéant, un trajet de post-réaction.

8. Procédé suivant l'une des revendications 1 à 7, caractérisé en ce que le catalyseur, separé du produit réactionnel, est recyclé dans le procédé, le cas échéant après addition d'un dérivé de métal alcalin ou alcalinoterreux basique frais.